# EUROPÄISCHE PATENTSCHRIFT

(11) **EP 1 787 607 B1**
(45) Veröffentlichungstag und Bekanntmachung des Hinweises auf die Patenterteilung: **09.11.2011**
(21) Anmeldenummer: 05025133.9
(22) Anmeldetag: 17.11.2005
(51) Int. Cl.: A61F 9/01

(54) **Anordnung zur Durchführung chirurgischer Laserbehandlungen des Auges**
Apparatus for surgical laser treatments of the eye
Dispositif de traitement chirurgicale de l'oeil

(43) Veröffentlichungstag der Anmeldung: 23.05.2007
(73) Patentinhaber: WaveLight GmbH, 91058 Erlangen (DE)
(72) Erfinder: König, Karsten, Prof. Dr., 66119 Saarbrücken (DE); Le Harzic, Ronan, Dr., 57200 Sarreguemines (FR); Wüllner, Christian, 91096 Möhrendorf (DE); Donitzky, Christof, 90542 Eckental (DE)
(74) Vertreter: von Hellfeld, Axel

(56) Entgegenhaltungen:
- EP-A- 1 537 841
- US-A- 5 656 186
- US-A1- 2004 199 149
- US-A1- 2005 085 800
- US-E1- R E37 504

## Beschreibung

Die Erfindung betrifft eine Anordnung und ein Verfahren zur Durchführung chirurgischer Laserbehandlungen der Augenhornhaut.

Es ist im Stand der Technik bekannt, sogenannte Femtosekundenlaser, also gepulste Laser mit Pulsdauern im Femtosekundenbereich, für die ophthalmologische Chirurgie einzusetzen, insbesondere zur Trennung von Gewebestrukturen am oder im Auge. Zum Beispiel werden mit Femtosekundenlasern sogenannte Flap-Schnitte ausgeführt, also Einschnitte in das Auge von der Seite, um einen kleinen Deckel ("Flap") zu erzeugen, der für z.B. einen LASIK-Eingriff zur Seite geklappt wird. Auch in der sogenannten Keratoplastik finden Femtosekundenlaser Verwendung.

Bisher in der ophthalmologischen Chirurgie verwendete Femtosekundenlaser emittieren in der Regel Strahlung mit Wellenlängen im IR-Bereich oder im sichtbaren Bereich des elektromagnetischen Spektrums.

US 5,984,916 befasst sich mit der kornealen und intraokularen Laserchirurgie, wobei gepulste Laserstrahlung zum Einsatz kommt, deren Pulsdauer zwischen 10 fs und 2 ps bei einer Wellenlänge von etwa 400 nm aufwärts liegt.

US 5,656,186 beschreibt eine Methode zur Bestimmung einer geeigneten Pulsdauer für die Bearbeitung von Materialien unterschiedlichster Art mit gepulster Laserstrahlung. Gemäß dieser Methode wird zunächst experimentell bei einer gegebenen Wellenlänge die Abhängigkeit der Schwelle für den optischen Durchbruch des Materials von der Pulsdauer ermittelt. Sodann wird in einer die gefundene Abhängigkeit beschreibenden Kurve ein Punkt ermittelt, ab dem zu kürzeren Pulsdauern hin eine quadratische Abhängigkeit der Durchbruchschwelle von der Pulsdauer nicht mehr gilt. Die eigentliche Laserbearbeitung wird dann mit einer Pulsdauer jenseits dieses Übergangspunkts durchgeführt. Hierfür werden Pulsdauern im Femto- und Pikosekundenbereich angegeben.

Die in US 5,656,186 beschriebene Methode soll anwendbar sein in einem äußerst breiten Wellenlängenbereich, der nahezu das gesamte praktisch genutzte Laserspektrum abdeckt. Jedenfalls ist in dieser Schrift ein Wellenlängenbereich von 200 nm bis hin zu 2 µm erwähnt. Als Beispiele für die Materialvielfalt, bei der die Methode anwendbar sein soll, werden Metalle wie Gold oder Aluminium, Glaswerkstoffe, aber auch lebendes Gewebe genannt, insbesondere die Kornea. Für die Laserbehandlung der Kornea ist konkret eine Wellenlänge von 770 nm angegeben.

US 2004/0199149 A1 beschreibt die Verwendung von fs-Laserstrahlung zur Bearbeitung der Linse des menschlichen Auges unter anderem zur refraktiven Sehfehlerkorrektur. Für die Behandlungswellenlänge werden Bereiche von 310 bis 350 nm und 700 bis 1500 nm vorgeschlagen, die Pulsenergie soll zwischen 0,1 mJ und 10 mJ liegen.

EP 1 537 841 A2 beschreibt die Verwendung von fs-Laserstrahlung zur Bearbeitung von Trübungen oder Verhärtungen in der Hornhaut, der Linse oder anderer Teile des menschlichen Auges. Die Behandlungswellenlänge soll bei 350 nm oder darüber liegen, die Pulsenergie größer 1 mJ sein.

US RE37,540 E offenbart eine Laservorrichtung zur Behandlung der Augenhornhaut. Dort werden für die korneale Photoablation Wellenlängen zwischen 190 und 220 nm bei Pulsdauern bis hin in den Sub-Pikosekundenbereich erwähnt. Die Pulsenergie liegt in einem Bereich von zweistelligen Mikrojoule bis hin zu einigen Millijoule.

US 2005/085800 A1 offenbart ein Lasersystem zur refraktiven Laserchirurgie beispielweise der Kornea. Das Lasersystem umfasst zwei gesonderte Laserquellen, die sich einen gemeinsamen Scanner teilen und wechselweise betreibbar sind. Die Laserquellen werden von einer gemeinsamen Pumpquelle gepumpt. Beide enthalten ein bei 840 nm emittierendes Ti:Sa-Lasermaterial. Durch Frequenzkonversion wird die Wellenlänge des Lasermaterials einer der Laserquellen auf etwa 200 nm umgewandelt. Die andere Laserquelle erzeugt fs-Strahlung mit Impulsenergien von einigen µJ.

Der Erfindung liegt die Aufgabe zugrunde, eine Anordnung zur Durchführung chirurgischer Laserbehandlungen der Augenhornhaut bereitzustellen, mit der verbesserte Operationsergebnisse erreichbar sind. Insbesondere soll es die Anordnung ermöglichen, unerwünschte Änderungen in den Gewebestrukturen des Auges besser vermeiden zu können, wie zum Beispiel Schädigungen der Linse oder der Retina bei Eingriffen in die Kornea.

Zur Lösung dieser Aufgabe ist erfindungsgemäß eine Anordnung mit den Merkmalen des Anspruchs 1 vorgesehen. Bevorzugte Weiterbildungen sind in den abhängigen Unteransprüchen angegeben.

Der Erfindung liegt die Erkenntnis zugrunde, dass bei Einwirkung gepulster Behandlungsstrahlung mit einer Wellenlänge zwischen etwa 340 nm und etwa 360 nm, einer Pulsdauer im Femtosekundenbereich und einer Pulsenergie der Behandlungsstrahlung zwischen etwa 0,1 und etwa 5 µJ unerwünschte Beeinträchtigungen von Augenbereichen, die nicht durch die elektromagnetische Strahlung bei der Operation beeinflusst werden sollen, besser vermieden werden können.

Hingegen können bei Verwendung von Strahlung im Infrarot-Bereich oder auch im sichtbaren Bereich derartige Schädigungen nicht (immer) ausgeschlossen werden.

Bevorzugt weist die erfindungsgemäße Anordnung Mittel zur Fokussierung der Behandlungsstrahlung auf oder in einer Kornea auf. Damit ist es möglich, nur die Kornea chirurgisch zu beeinflussen, insbesondere durch Photoablation oder/und - disruption, etwa um oberflächlich oder intrageweblich Hornhautmaterial abzutragen und/oder um Schnitte in der Hornhaut anzubringen, beispielsweise zur Erzeugung eines Hornhautscheibchens (Flap) im Rahmen einer LASIK-Behandlung. Dabei kann vermieden werden, dass andere Strukturen des Auges, wie zum Beispiel die Linse und/oder die Retina, in erheblicher Weise getroffen werden.

Gemäß einer bevorzugten Ausgestaltung der Erfindung liegt die Wellenlänge der emittierten Behandlungsstrahlung bei etwa 347 nm. Letztere Wellenlänge ist beispielsweise durch Frequenzverdreifachung aus einer Grundwellenlänge von 1040 nm erzeugbar. Der Fachmann ist mit Konzepten zur Frequenzkonversion vertraut, weswegen auf Einzelheiten der Frequenzwandlung hier nicht näher eingegangen werden muss. Geeignete Wandler können beispielsweise mit Komponenten wie optisch parametrischen Generatoren/Oszillatoren, Summenfrequenzgeneratoren und Generatoren für die zweite, dritte, vierte oder fünfte Harmonische aufgebaut werden. Femtosekundenlaser, welche die vorstehende Grundwellenlänge und auch andere Wellenlängen im kurzwelligen Infrarotbereich emittieren, sind handelsüblich erhältlich.

Es hat sich gezeigt, dass bei Verwendung von Laserpulsen der genannten Wellenlängen und Pulsdauern im Femtosekundenbereich zur Korneabehandlung, etwa zur Erzeugung eines Flaps, nahezu die gesamte eingestrahlte Energie in der Kornea umgesetzt wird und etwaige als Resttransmission durch die Kornea tretende Strahlung von der humanen Linse und nicht der Retina absorbiert wird.

Infolge relativ hoher Pulsrepetitionsraten kann auch die sogenannte Ablationsschwelle gesenkt werden, ab der - statistisch betrachtet - Photoablation in signifikantem Maß auftritt. Bei der Erfindung wird deshalb vorzugsweise eine Pulsrepetitionsrate der Behandlungsstrahlung von wenigstens etwa 10 kHz gewählt, vorzugsweise zwischen etwa 100 kHz und etwa 500 kHz. Selbstverständlich kann auch eine Pulsrepetitionsrate bis hin in den MHz-Bereich gewählt werden, sogar beispielsweise im zweistelligen MHz-Bereich.

Die Pulsenergie der Behandlungsstrahlung beträgt vorzugsweise höchstens etwa 10 nJ. Beispielsweise können Pulsenergien von nicht mehr als 0,8 nJ pro Puls, insbesondere nicht mehr als 0,7 nJ pro Puls und weiter insbesondere von nicht mehr als 0,5 nJ pro Puls eingesetzt werden. Die minimalen Energiegrenzwerte hängen dabei von der Repetitionsrate und der numerischen Apertur ab und sind jeweils so einzustellen, dass der gewünschte Effekt eintritt, also zum Beispiel die Ablationsschwelle erreicht wird.

Als Beispiel ermöglicht die Erfindung die Erzeugung eines Hornhautflaps höchster Qualität für die LASIK-Operation mit gepulster UV-Strahlung mit nur 0,5 nJ/Puls. Eine Zeitspanne von 30 Sekunden bis 1 Minute für die Erzeugung des Flaps ist dabei erreichbar.

Der Begriff Femtosekundenbereich soll im Rahmen der Erfindung auch Pulslängen von mehreren hundert fs erfassen, also zum Beispiel Pulslängen mit 230 fs Halbwertslänge. Insbesondere soll dieser Begriff auch Pulslängen im einstelligen Pikosekundenbereich bis hin zu etwa 10 ps erfassen. Zum Beispiel können die Pulslängen bei FS-Lasern auf 1 ps durch Verwendung geeigneter optischer Mittel gestreckt werden.

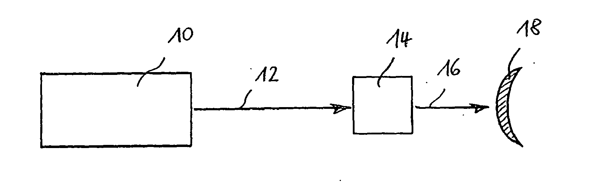

Nachfolgend wird mit Blick auf die beigefügte einzige Figur ein Ausführungsbeispiel der Erfindung beschrieben. Die Figur zeigt schematisch ein Laserbehandlungssystem mit einem gepulsten Femtosekundenlaser 10.

Der Laser 10 stellt durch Erzeugung der dritten Harmonischen aus einer Grundwellenlänge von 1040 nm eine Wellenlänge von 347 nm bereit. Die emittierten Strahlungspulse 12 haben Pulslängen von 250 fs (FWHM) bei einer Repetitionsrate von 20 MHz. Die Leistung liegt bei 27 mW. Mittel zur Strahlformung und -führung sind mit dem Block 14 pauschal angedeutet und als solches dem Fachmann bekannt. Diese Mittel sind insbesondere dazu eingerichtet, die Strahlungspulse so zu lenken, dass der gesamte Zielbereich überstrichen wird. Auch sind die Mittel 14 mit einer Fokussierfunktion ausgestattet, die es gestattet, die Strahlungspulse auf einen gewünschten Punkt zu fokussieren, der beispielsweise an der Oberfläche des Zielgewebes oder in dessen Tiefe liegen kann. Die so durch die Mittel 14 in Zeit und Raum gesteuerten Strahlungspulse, nunmehr mit 16 bezeichnet, werden im dargestellten Beispielfall auf eine Kornea 18 gerichtet, etwa um ein Flap zu erzeugen.

Die Mittel 14 zur Strahlungsformung und -führung können gewünschtenfalls eine Objektivanordnung enthalten. Eine solche ist jedoch im Rahmen der Erfindung nicht notwendig.

Gute Ablationsergebnisse können beispielsweise bei einer Wellenlänge von 347 nm mit Repetitionsraten im Bereich von 20 MHz, einer maximalen Energie von 1 nJ pro Puls und unter Verwendung eines UV-Fokussierungsobjektivs (100X) erreicht werden. Dies entspricht etwa einer Fluence von 2 J/cm².

Mit den vorstehend genannten Parametern ist eine im wesentlichen vollständige Strahlungsabsorption in der Kornea möglich bei gleichzeitig äußerst geringer Strahlungspenetration in andere Augenbereiche. Mit sehr geringer Pulsenergie können qualitativ hochwertige Flaps in einer akzeptablen Zeit von weniger als 1 Minute erzeugt werden.

## Patentansprüche

1. Anordnung zur Durchführung chirurgischer Laserbehandlungen der Augenhornhaut, wobei die Anordnung dazu eingerichtet ist, gepulste Behandlungsstrahlung mit einer Wellenlänge zwischen 340 nm und 360 nm und einer Pulsdauer im Femtosekundenbereich zu emittieren, wobei die Pulsenergie der Behandlungsstrahlung zwischen 0,1 nJ und 5 µJ liegt, vorzugsweise höchstens 10 nJ beträgt.

2. Anordnung nach Anspruch 1,
**gekennzeichnet durch** Mittel zur Fokussierung der Behandlungsstrahlung auf oder in einer Kornea.

3. Anordnung nach Anspruch 1 oder 2,
**dadurch gekennzeichnet, dass** die Wellenlänge der emittierten Behandlungsstrahlung bei 347 nm liegt.

4. Anordnung nach einem der vorhergehenden Ansprüche,
**dadurch gekennzeichnet, dass** die Pulsrepetitionsrate der Behandlungsstrahlung wenigstens 10 kHz beträgt, vorzugsweise zwischen 100 kHz und 500 kHz liegt.

## Claims

1. Apparatus for performing laser surgical treatments of the cornea of an eye, the apparatus configured to emit pulsed treatment radiation having a wavelength between 340 nm and 360 nm and a pulse duration in the femtosecond region, the pulse energy of the treatment radiation being between 0,1 nJ and 5 µJ, preferably no more than 10 nJ.

2. Apparatus of claim 1, **characterized by** means for focusing the treatment radiation onto or into a cornea.

3. Apparatus of claim 1 or 2, **characterized in that** the wavelength of the emitted treatment radiation is at 347 nm.

4. Apparatus of any one preceding claim, **characterized in that** the pulse repetition rate of the treatment radiation is at least 10 kHz, preferably between 100 kHz and 500 kHz.

## Revendications

1. Dispositif pour le traitement chirurgical par laser de la cornée de l'oeil, ledit dispositif étant aménagé pour émettre un rayonnement traitant pulsé dans une plage de longueur d'onde comprise entre 340 nm et 360 nm et d'une durée d'impulsion de l'ordre de la femtoseconde, l'énergie d'impulsion du rayonnement traitant étant comprise entre 0,1 nJ et 0,5 µJ, de préférence de 10 nJ au maximum.

2. Dispositif selon la revendication 1,
**caractérisé par** des moyens permettant la focalisation du rayonnement traitant sur ou dans une cornée.

3. Dispositif selon la revendication 1 ou 2,
**caractérisé en ce que** la longueur d'onde du rayonnement traitant émis est de 347 nm.

4. Dispositif selon l'une des revendications précédentes,
**caractérisé en ce que** la fréquence de répétition des impulsions du rayonnement traitant est au moins de 10 kHz, de préférence comprise entre 100 kHz et 500 kHz.
